# EUROPEAN PATENT APPLICATION

(11) **EP 2 839 852 A1**
(43) Date of publication of application: **25.02.2015**
(21) Application number: 13778642.2
(22) Date of filing: 19.04.2013
(51) Int. Cl.: A61M 3/02, A61M 11/00, A61H 35/04

(54) **WASHING WATER SPRAYING VESSEL FOR NASAL CAVITY CLEANER**

(30) Priority: 20.04.2012 KR 20120041276
(71) Applicant: Yonwoo Co., Ltd., Incheon 404-250 (KR)
(72) Inventor: KIM, Sung-Hwan, Incheon 404-250 (KR); KIM, You-Seob, Incheon 404-250 (KR)
(74) Representative: Patentanwälte ter Smitten Eberlein Rütten Partnerschaftsgesellschaft
(86) International application number: PCT/KR2013/003327
(87) International publication number: WO 2013/157878

(57) **Abstract**

The present invention relates to a washing water spraying vessel for a nasal cavity cleaner providing a locking function by means of a one-way rotation of an outer cap, thereby completely preventing the outer cap from being pressed due to external pressure when being carried, and thus can prevent unnecessary waste of the washing water, as well as prevent contamination of the interior of an overcap

## Description

### FIELD OF TECHNOLOGY

The present invention relates to a washing water spraying vessel for a nasal cavity cleaner, specifically providing a locking function by means of a one-way rotation of an outer cap, thereby completely preventing the outer cap from being pressed due to external pressure when being carried, and thus can prevent unnecessary waste of the washing water, as well as prevent contamination of the interior of an overcap.

### BACKGROUND OF THE INVENTION

Generally, washing water spraying vessels for a nasal cavity cleaner are containers for curing all kinds of the diseases in a nasal cavity or eliminating impurities, and preventing dryness by inserting washing water into the nasal cavity, and it is said that the nasal cavity comprises the front unit connected to the outside through nares, and the rear unit connected to a pharynx through a posterior nares.

The washing water inserted into the above-mentioned washing water spraying vessel for a nasal cavity cleaner generally uses a 3% saline solution, and recently functional washing water containing various ingredients such as bamboo salt is being improved.

In addition, lately, air pollution caused by exhaust gas from vehicles, pollen and yellow dust in the spring, and cold viruses prevailing during in-between seasons are inhaled through a mouth and a nose, thereby causing harmful materials for the human body to be deposited or various respiratory diseases and hay fever or colds to be developed with frequency; whereupon, there are an increasing number of people who suffer from snoring.

It is already proved through many medical reports that washing the nasal cavity is effective for all kinds of the respiratory diseases, and patients who suffer from all kinds of the respiratory diseases wash their nasal cavity several times a day.

With reference to Utility Model Registration No. 20-0165667, a conventional washing water spraying vessel for a nasal cavity cleaner is described below. Referring to the registered utility model, the conventional washing water spraying vessel for a nasal cavity cleaner comprises a container 100 receiving a cleaning liquid, a lid 200 screw-coupled in a spout portion of the container 100, a nozzle 300 vertically movably coupled to the lid 200, wherein a hanging part 310 for allowing vertically moving a finger to hang is formed in a lower part, a bottom liquid chamber 320 is formed in an inner top, and a spurting opening 321 for spurting the cleaning liquid in an upper part, an adjustment rod 330 inserted into the bottom liquid chamber 320 of the nozzle 300, for uniformly and thinly spraying the cleaning liquid, and valves 550, 551 for spurting through the spurting opening 321 the cleansing liquid after the cleansing liquid included in the container 100 is stored in the bottom liquid chamber 310 by vertically moving the nozzle 300.

In the washing water spraying vessel for a nasal cavity cleaner consisting of the above-described configuration, it is configured that the nozzle 300 is coupled to the lid 200 of the container 100 to be upwardly slid, so that the cleansing liquid is spurted by the operation of the valves 550, 551 of an inner side according to repeatedly perform pressing by putting the finger in the hanging part 310, thereby easily portable to use.

However, the above-mentioned washing water spraying vessel for a nasal cavity cleaner does not have a structure that limits the movement of a latch, so that, while in the bag, though a user does not manipulate it, the latch 310 is pressed and spurts washing water. As a result, washing water can be spilled by pressing the latch 310, and thus, not only may the content be unnecessarily consumed but the inside of the overcap may be contaminated.

### SUMMARY OF THE INVENTION

The present invention is devised to solve the above mentioned problems; the purpose of the present invention is to provide a washing water spraying vessel for a nasal cavity cleaner having a locking function by means of a one-way rotation of an outer cap, thereby completely preventing the outer cap from being pressed due to external pressure when being carried, and thus can prevent unnecessary waste of the washing water, as well as prevent contamination of the interior of an overcap

To solve the above problems, a washing water spraying vessel for a nasal cavity cleaner according to the present invention comprises: a container body wherein content are stored; an inner cap which is engaged on the inner side, covering the upper portion of the container body so as to be rotated, with a pump-blocking projection protruded on the side; an outer cap which is coupled, covering the upper portion of the inner cap, and can move upwards and downwards according to the location of the pump-blocking projection upon the rotation; a pumping member which discharges the content through pumping operation by the upward and downward movement of the outer cap; and an overcap which is coupled, covering the outer cap.

In addition, the outer cap comprises: a body which is coupled, covering the upper portion of inner cap, further comprising an insertion groove, wherein a part of the lower portion is cut open and the pump-blocking projection is inserted according to the rotation; and a combining pipe which is coupled with protrusion from the center of the upper portion of the body and combined with the pumping member, pressurizing the pumping member.

In addition, on the inner circumference surface is installed a pumping guide groove, wherein the pump-blocking projection is inserted. The length of the pumping guide groove should be the same or longer than the length of the pump-blocking projection so as not to cause inference with the pump-blocking projection according to the descent of the body.

In addition, the pumping guide groove comprises a gentle slanting surface with one side so as not to cause interference with the pump-blocking projection when the outer cap is rotated.

In addition, on the inner circumference surface of the outer cap is installed a rotation display part, which comprises a gentle slanting surface so as not to cause interference with the pump-blocking projection when the outer cap is rotated.

In addition, on the outer circumference surface of the outer cap is installed a rotation display part showing the direction of rotation.

In addition, on the upper portion of the inner cap is installed an engaging protrusion, which limits the ascent of the outer cap, and a protrusion is formed on the inner side of the outer cap, where the protrusion meets the engaging protrusion.

As the above mentioned, according to the present invention, by providing a locking function by means of a one-way rotation of an outer cap, thereby completely preventing the outer cap from being pressed due to external pressure, the present invention thus has an advantage that it can prevent unnecessary waste of the washing water, as well as prevent contamination of the interior of an overcap

### BRIEF DESCRIPTION OF THE DRAWINGS

FIG. 1 is a perspective view illustrating a configuration of a washing water spraying vessel for a nasal cavity cleaner according to an exemplary embodiment of the present invention;
FIG. 2 is an exploded perspective view illustrating a configuration of a washing water spraying vessel for a nasal cavity cleaner according to an exemplary embodiment of the present invention;
FIG. 3 is a cross-sectional view illustrating a configuration of a washing water spraying vessel for a nasal cavity cleaner according to an exemplary embodiment of the present invention;
FIGS. 4 and 5 are views illustrating a position of the pump-blocking projection by the rotation of an outer cap of a washing water spraying vessel for a nasal cavity cleaner according to an exemplary embodiment of the present invention.
FIG. 6 is a view illustrating discharging process by upward and downward movement of the outer cap of a washing water spraying vessel for a nasal cavity cleaner according to an exemplary embodiment of the present invention.

### DETAILED DESCRIPTION OF PREFERRED EMBODIMENTS

Hereinafter, exemplary embodiments of the present invention will be described in detail with reference to the accompanying drawings. The same reference numerals provided in the drawings indicate the same members.

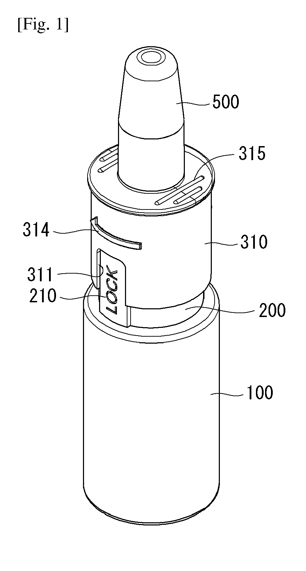

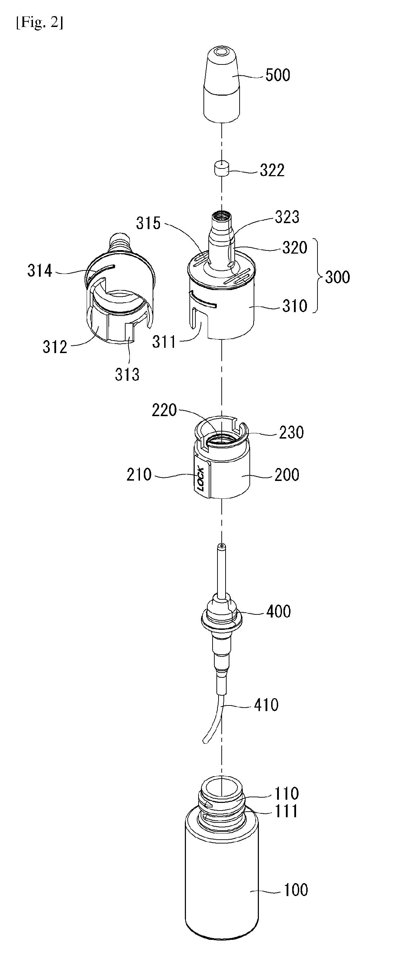

FIG. 1 is a perspective view illustrating a configuration of a washing water spraying vessel for a nasal cavity cleaner according to an exemplary embodiment of the present invention. FIG. 2 is an exploded perspective view illustrating a configuration a washing water spraying vessel for a nasal cavity cleaner according to an exemplary embodiment of the present invention.

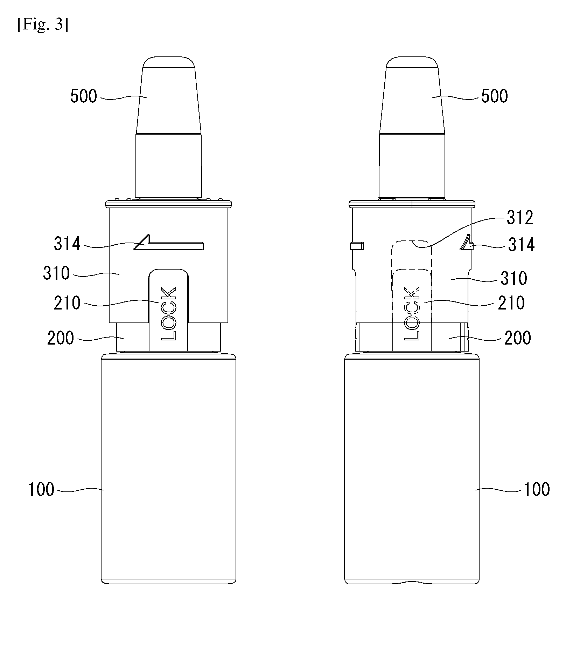

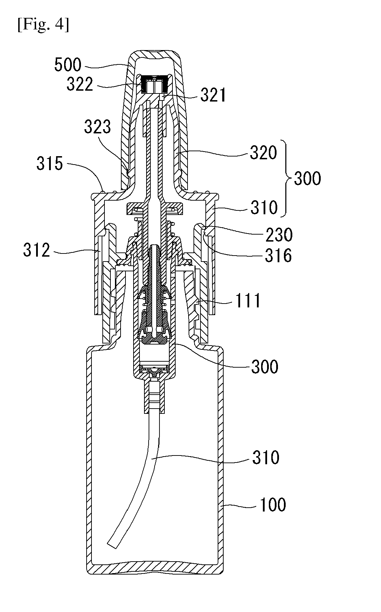

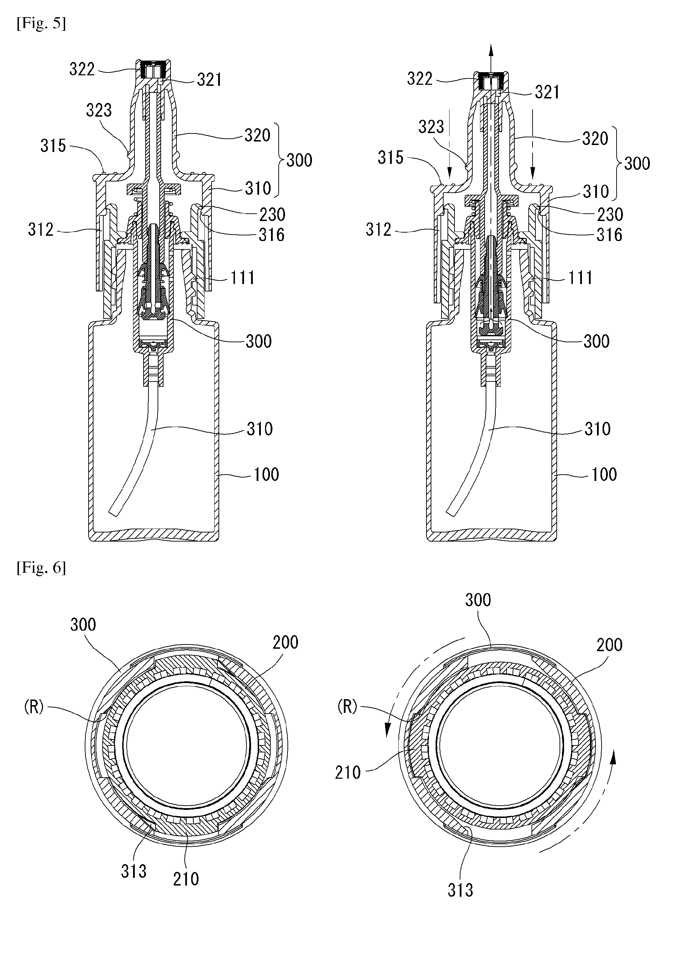

FIG. 3 is a cross-sectional view illustrating a configuration of a washing water spraying vessel for a nasal cavity cleaner according to an exemplary embodiment of the present invention. FIGS. 4 and 5 are views illustrating a position of the pump-blocking projection by the rotation of an outer cap of a washing water spraying vessel for a nasal cavity cleaner according to an exemplary embodiment of the present invention.

Referring to FIGS. 1 to 6, a washing water spraying vessel for a nasal cavity cleaner according to an exemplary embodiment of the present invention comprises a container body 100, an inner cap 200, an outer cap 300, a pumping member 400, and an over cap 500.

Content is contained in the container body 100, on the upper part of which is installed a discharging unit 110 which lead content to flow out. On the outer circumference of the discharging unit 110 is installed 1^{st} screw thread 111 which can be engaged and screwed up with an inner cap 200.

The inner cap 200 is engaged with the discharging unit 110, wrapping the upper part of the container body 100, and on the inner circumferential surface is formed 2nd screw thread 220, to be able to be engaged and screwed up with the 1^{st} screw thread 111.

As for this present invention, it is featured that on the side of the said inner cap 200 is installed a pump-blocking projection 210 blocking the downward movement of the outer cap 300 is engaged with protrusion. When a pair of said pump-blocking projection 210 is formed with protrusion in outer direction and is inserted into an insertion groove 311 by the rotation of the outer cap 300, pumping action does not occur and thus prevent content from being discharged.

It is preferred that a latching device is displayed on the said pump-blocking projection 210, so that a user can notice whether a pumping member 400 is in the state of being operated or not.

Meanwhile, it is preferred that an engaging protrusion 230, which limits the ascent of the outer cap 300, is built on the upper part of the inner cap 200 the outer cap 300 in order to prevent the outer cap 300 from moving upwards and being separated.

The said outer cap 300 which encloses the upper part of the inner cap 200 and is engaged in order be rotated, is moved upward and downwards by the pressure given by a user, and delivers pressure to the pumping member 400, comprising a container body 310 and a combining pipe 320.

The said container body 310 encloses the upper part of the inner cap 200, and it is featured that in this present invention, an insertion groove 311 is installed on the said container body 310 with a part of the bottom cut and the said pump-blocking projection 210 inserted by the rotation.

When the pump-blocking projection 210 is inserted by the rotation of the outer cap 300, the said insert groove 311 is built to be contacted with the upper end of the pump-blocking projection 210 and keeps its locked state, when pumping action does not occur. It is preferred that the said insertion groove 311 should be composed of a pair so that the pair inserted respectively can face each other.

In addition, on the inner circumference surface is installed a pair of pumping guide grooves 312 wherein the said pump-blocking projection 210 is inserted; when the said pumping guide groove 312 rotates the outer cap 300 by 90 degrees in the locked state, the pump-blocking projection 210 is inserted into; hence, it is preferred that the length of the pumping guide groove 312 is the same or longer than the length of the pump-blocking projection 210 in order that the pumping groove 312 can perform pumping operation without interference with pump-blocking projection 210 by the up and down movement of the outer cap 300 when the pump-blocking projection 210 is inserted.

The said pumping guide groove 312 is installed with a gentle slanting surface on one side, in order not to cause interference with the said pump-blocking projection 210 when the outer cap 300 is rotated; on the other hand, it is preferred that the other side of the pumping guide groove 312 has a right angled surface in order for the outer cap 300 not to turn in a reverse direction.

It is featured that the outer cap 300 is supposed to turn to only one direction, namely to the closing direction of the inner cap 200; whereupon, it can prevent the separation of the screw joint, between the container body 100 and the inner cap, 200, which may be caused by the reverse rotation of the outer cap 300.

Furthermore, It is recommended that a rotation guide projection 313 be installed on the outer surface of the said body 310 which forms a gentle slanting surface so that the outer cap 300 can be rotated easily without interference of the pump-blocking projection 210, when the pump-blocking projection 210 is inserted into the insertion groove 311 and locked in place, and then rotates the outer cap 300.

Furthermore, it is recommended that a rotation display part 314 be installed on the outer surface of the body 310 so that a user may be able to notice easily the rotating direction of the outer cap 300.

Furthermore, it is recommended that, on the inner surface of the outer cap 300, protrusion 316 is installed on the area on which the said engaging protrusion 230 is contacted in order to prevent the outer cap 300 from being separated from the inner cap 200.

The said combining pipe 320 is installed with protrusion upwards from the upper center of the said body 310, and, in its inside, is connected with the upper part of the pumping member 400, so that the outer cap 300 is descended by a user's pressing and delivers the pressure to the pumping member 400, which causes the pumping movement of the pumping member 400.

On the upper end of the combining pipe 320 is installed a discharging hole 321 which discharge content by the pumping movement of the pumping member 400, whereas on the upper part is installed a nozzle tip which discharges content to the outside.

The said pumping member 400 is engaged into the inner surface of the said outer cap 300 and discharge content through pumping caused by the ascent and descent movement of the outer cap 300, wherein on the lower end of the container body 100 is engaged a suction pipe 410 which aspirates content contained in the container body 100. Each function and construction of the pumping member 400 is a prior art of technological field where the present invention belongs to; therefore detailed description will be omitted.

The said overcap 500 is engaged, encircling the said outer cap 300, and is connected with a combining projection 323 installed in the lower part of the combining projection 320, protecting a nozzle tip 322 from the impact.

Hereinafter, an operation process of a washing water spraying vessel for a nasal cavity cleaner according to an exemplary embodiment of the present invention will be described with reference to FIGS. 4 and 6.

FIGS. 4 and 5 are views illustrating an operation state of a washing water spraying vessel for a nasal cavity cleaner according to an exemplary embodiment of the present invention. FIG. 6 is a view illustrating discharging process of the content by descent and ascent movement of the outer cap in a washing water spraying vessel for a nasal cavity cleaner according to an exemplary embodiment of the present invention.

Referring to FIG. 4 to 6, a washing water spraying vessel for a nasal cavity cleaner according to an exemplary embodiment of the present invention is firstly that the pump-blocking projection 210 of the inner cap 200 rotates the outer cap 300 as much as 90 degree under the state that it is inserted and latched into the insertion hold 311 of the outer cap 300, when the pump-blocking projection 210 is inserted into the pumping guide groove 312. At this time, the outer cap 300 is formed to be rotated without interference with the pump-blocking projection 210 by the rotation guide projection 313.

As above, when the pumping guide projection 210 is inserted to the pumping guide groove 312, locking state is released and allows the outer cap 300 to ascend or descend, and then pumping action by the pumping member 400 is generated, causing the content discharged. At this time, the length of the said pumping guide groove 312 is preferred to be the same or longer length than the length of the pump-blocking projection 210 in order that the pumping guide groove 312 does not cause interference by the pump-blocking projection 210 when the outer cap 300 ascends or descends.

The best embodiments of the present invention are described with reference to the drawings and the specification. Here, even though particular terms are used, they are used to explain the present invention, not to limit their meanings or the range of the present invention described in the claims. Therefore, it will be understood by those skilled in the art that various changes in form and other equivalent embodiments may be made therefrom. Accordingly, the truly technical protection scope of the present invention is to be determined by the technical spirit of the appended claims.

## Claims

1. A washing water spraying vessel for a nasal cavity cleaner, comprising:
a container body containing content;
an inner cap which is engaged to an upper portion of the container body, while covering the upper portion of the container body, wherein a pump-blocking projection is coupled with protrusion on the side of the container body;
an outer cap which is coupled to be able to rotate, while covering an upper portion of the inner cap, and ascends and descends upon the location of the pump-blocking projection by the rotation;
a pumping member discharging the content to the outside through pumping operation by the up and down movement of the outer cap;
an overcap which is combined to the upper portion of the outer cap, while covering the upper portion of the outer cap.

2. The washing water spraying vessel for a nasal cavity cleaner of claim 1, wherein the outer cap comprises:
a body comprising an insertion groove which is coupled, while covering the upper portion of the inner cap, with a part of the lower end cut and the pump-blocking projection inserted by the rotation; and
a combining pipe coupled with upward protrusion from the central portion of the upper end of the body, and combined with the pumping member, thereby pressuring the pumping member.

3. The washing water spraying vessel for a nasal cavity cleaner of claim 2, wherein a pumping guide groove, into which a pump-blocking projection is inserted, is installed on the inner circumference surface of the body, and further, wherein the length of the pumping guide groove is the same or longer than the length of the pump-blocking projection in order not to cause interference with the pump-blocking projection upon the descent of the body.

4. The washing water spraying vessel for a nasal cavity cleaner of claim 3, wherein the pumping guide groove comprises a gentle slanting surface on one side, in order not to cause interference with the pump-blocking projection when the outer cap 300 is rotated.

5. The washing water spraying vessel for a nasal cavity cleaner of claim 2, wherein a rotating guide projection comprises a gentle slanting surface on the inner circumference surface of the body, so as not to cause interference with the pump-blocking projection when the outer cap 300 is rotated.

6. The washing water spraying vessel for a nasal cavity cleaner of claim 1 comprises a rotation display part, which indicates the direction of rotation, on the outer circumference surface.

7. The washing water spraying vessel for a nasal cavity cleaner of claim 1, comprising:
an engaging protrusion limiting the ascent of the outer cap on the upper portion of the inner cap; and
a protrusion coupled on the part where the protrusion meets the engaging protrusion on the inner side of the outer cap.
